# EUROPEAN PATENT SPECIFICATION

(11) **EP 4 155 205 B1**
(45) Date of publication and mention of the grant of the patent: **27.11.2024**
(21) Application number: 21198904.1
(22) Date of filing: 24.09.2021
(51) Int. Cl.: B64D 11/00, B60N 2/00

(54) **SYSTEM FOR IN-FLIGHT DETECTION OF PHYSIOLOGICAL DATA AND KIT FOR IN-FLIGHT MONITORING OF PHYSIOLOGICAL PARAMETERS**
SYSTEM ZUR ERFASSUNG VON PHYSIOLOGISCHEN DATEN WÄHREND DES FLUGES UND KIT ZUR ÜBERWACHUNG VON PHYSIOLOGISCHEN PARAMETERN WÄHREND DES FLUGES
SYSTÈME DE DÉTECTION DE DONNÉES PHYSIOLOGIQUES EN VOL ET KIT DE SURVEILLANCE DE PARAMÈTRES PHYSIOLOGIQUES EN VOL

(43) Date of publication of application: 29.03.2023
(73) Proprietor: Aircraft Cabin Modification GmbH, 87700 Memmingen (DE)
(72) Inventor: OEHLER, Martin, 38106 Braunschweig (DE); NOSHARI, Arash, 22605 Hamburg (DE); THAHER, Rami, 87700 Memmingen (DE)
(74) Representative: Vossius & Partner Patentanwälte Rechtsanwälte mbB

(56) References cited:
- EP-A1- 3 042 608
- JP-A- 2013 212 311
- US-A1- 2017 169 690

## Description

Air travel is widespread nowadays, being used frequently by a relatively large proportion of the world's population for private and/or business-related purposes. In general, air travel may be conducted in unpowered aircrafts, such as gliders or parachutes, and in powered aircrafts, such as propeller aircrafts and/or jet aircrafts.

In particular in powered airplanes and/or helicopters, air travel is considered to be a relatively fast and safe way of traveling for private and/or business-related purposes. Moreover, many people operate aircrafts, either powered and/or unpowered aircrafts, during their leisure time as a hobby.

Due to the large distances covered during many aircraft flights, i.e., long-distance flights, the in-flight time spent in the respective aircraft by the flight crew, e.g., the pilot(s) and/or flight attendants, and/or the passengers is often relatively long.

Hence, providing a comfortable, safe and/or appealing environment to the flight crew and the passengers during the respective aircraft flight is of utmost importance to the operators of aircrafts, in particular airline companies which operate commercial and/or cargo-carrying aircrafts.

For this reason, many in-flight amenities are provided by various aircraft operators, in particular airline companies. In many cases, the quality and the amount of the amenities have risen over the years.

Safety measures, for instance seat belts, are also provided by the operators of aircrafts, e.g., airline companies, to assist in providing a safe environment to the flight crew and the passengers in the aircraft, in particular in-flight.

Document EP3042608 discloses a seat configured so that the accuracy of detection of each sensor provided in the seat to detect the body potential of a seated occupant can be improved without worsening the sense of seating on the seat. The seat includes a trim cover having a contact surface for a seated occupant, and sensors arranged opposite to the contact surface and configured to detect the body potential of the seated occupant. Each sensor is a capacitive coupling sensor configured to detect the body potential through the trim cover. Moreover, a portion of the seat facing each sensor includes a dielectric configured to increase the dielectric constant of such a portion.

Document US2017169690 discloses an apparatus including an object for sitting or lying on, a plurality of sensors arranged within the object for sitting or lying on, and a signal processing unit connected to the sensors. The signal processing unit is provided to generate, on the basis of the sensor signals, an output signal which describes a posture and/or a movement of a person using the object for sitting or lying on. The generated output signal indicates a center of gravity of the person and/or a physiological process occurring in the person.

Document JP2013212311 discloses an electrocardiographic device for vehicles including a
direct electrode mounted on a steering wheel, a first capacitively coupled electrode and a second capacitively coupled electrode mounted on a backrest part of a seat and an electrocardiographic appliance. The first capacitively coupled electrode is mounted so as to be located at an upper and left side of the second capacitively coupled electrode when seen from a front side of the seat. The second capacitively coupled electrode is located at a low and right side relative to the first capacitively coupled electrode. The electrocardiographic appliance outputs a potential difference between a body potential detected by the direct electrode and a body potential detected by the second capacitively coupled electrode, filters electrocardiographic signals based on the potential difference and takes samples at a predetermined period to acquire an electrocardiographic wave form.

However, based on the prior art, there remains a need to improve the in-flight measures provided in aircrafts.

It is therefore an object of the present invention to provide an enhanced in-flight environment in aircrafts.

This object is achieved by a system for detecting physiological data of a person in-flight in an aircraft defined by the features of claim 1 according to a first aspect of the invention. Variations and further developments are defined by the features of the dependent claims.

The system includes at least one aircraft seat and/or at least one aircraft bed.

The aircraft seat, if present, may include a seating portion configured for the person, whose physiological data is to be detected, to at least partially sit on. The aircraft seat may also include a backrest configured for the person, whose physiological data is to be detected, to at least partially lean against, at least one armrest, and/or at least one headrest.

The aircraft seat may be configured to be fixedly mounted in an aircraft. For instance, the aircraft seat may be configured to be fixedly mounted, directly or indirectly, to a floor of the aircraft, e.g., by means of mounting bolts. Alternatively, or additionally, the aircraft seat may be configured to be connected to at least one adjacent aircraft seat and/or at least one adjacent aircraft seat.

The aircraft bed, if present, may include a lying portion configured for the person, whose physiological data is to be detected, to at least partially lie on, and optionally also at least one headrest.

The aircraft bed may be configured to be fixedly mounted in an aircraft. For instance, the aircraft bed may be configured to be fixedly mounted, directly or indirectly, to a floor of the aircraft, e.g., by means of mounting bolts. Alternatively, or additionally, the aircraft bed may be configured to be connected to at least one adjacent aircraft bed and/or at least one adjacent aircraft seat.

The system further includes at least one detecting device including at least one capacitive sensor integrated in the aircraft seat and/or the aircraft bed, respectively. The at least one detecting device may include a plurality of capacitive sensors. In other words, the aircraft seat and/or the aircraft bed may each include a plurality of capacitive sensors arranged in or on a portion thereof.

Preferably, the seating portion and/or the backrest and/or the armrest(s) and/or the headrest(s) of the aircraft seat each include a capacitive sensor. In the case of the aircraft bed, preferably the lying portion and/or the headrest(s) each include a capacitive sensor.

The capacitive sensor may be arranged on an outer surface of the aircraft seat, e.g., on an outer surface of the seating portion and/or the backrest and/or the armrest(s) and/or the headrest(s), or on an outer surface of the aircraft bed, e.g., on an outer surface of the lying portion and/or the headrest(s).

Additionally, or alternatively, the capacitive sensor may be arranged within the aircraft seat and/or aircraft bed, e.g., within a cushion of the seating portion and/or the backrest and/or the armrest(s) and/or the headrest(s) of the aircraft seat and/or within a cushion of the lying portion and/or the headrest(s) of the aircraft bed. The capacitive sensor may also be arranged underneath an outer surface of the aircraft seat and/or the aircraft bed, such as underneath a seat cover or bed cover, e.g., a seat and/or bed upholstery.

For instance, at least a first capacitive sensor may be arranged on an outer surface of the aircraft seat and/or the aircraft bed, as described above, and at least a second capacitive sensor may be integrated within at least one cushion and/or underneath at least one cover of the aircraft seat and/or the aircraft bed, as described above.

In general, the capacitive sensor(s) may be arranged on or in the aircraft seat and/or aircraft bed such that the capacitive sensor(s) is/are arranged proximate to the person's body, when the person is seated in the aircraft seat and/or is lying on the aircraft bed.

The aircraft seat and/or the aircraft bed may include at least one accessing element configured to allow access to the capacitive sensor(s) which is/are integrated in at least one portion of the aircraft seat and/or aircraft bed.

Thus, the accessing element may enable the capacitive sensor(s) to be accessed in order to, e.g., service the respective capacitive sensor and/or replace the respective capacitive sensor, e.g., when the capacitive sensor is faulty. This may facilitate servicing efforts of the system and thus reduce costs.

The accessing element may be, e.g., a removable portion of the aircraft seat and/or aircraft bed, e.g., a removable seat cover and/or bed cover, such as a removable seat upholstery and/or bed upholstery, or a removable portion of a cushion of the aircraft seat and/or aircraft bed.

The accessing element may also be a generally movable element, e.g., a swivelable and/or rotatable and/or slidable element, such as a door, flap or sliding element, which may be configured to allow access to the respective capacitive sensor by moving the movable element from a closed position, in which the movable element does not allow access to the respective capacitive sensor, to an access position, in which the movable element allows access to the respective capacitive sensor.

Existing aircraft seats and/or aircraft beds may be retrofitted with the capacitive sensor(s) to save costs when implementing the system described herein.

The capacitive sensor is configured to sense at least one physiological value of a person, when the person is arranged on at least a portion of the aircraft seat and/or aircraft bed.

The capacitive sensor may be configured to sense physiological value(s) of the person in a non-contact manner. In other words, the person's body does not have to be in contact with the capacitive sensor in order for the capacitive sensor to be able to sense the physiological value(s) of the person.

When the person's body is proximate to the capacitive sensor, e.g., when the person is seated in the aircraft seat and/or is lying on the aircraft bed, the person's body may interact with an electrostatic field generated by the capacitive sensor. This concept is generally known for, e.g., being used in capacitive input devices, i.e., touchscreens, and/or for sensing purposes, such as to sense the presence of an object.

As the person's body enters and/or moves in the electrostatic field generated by the capacitive sensor, a capacitance of the capacitive sensor is changed. This change in capacitance can be measured by the capacitive sensor.

Thus, movement of at least a portion of the person's body while sitting in the aircraft seat and/or while lying in the aircraft bed, such as movements caused by, but not limited to, the person's breathing and /or heart beat and/or blood pressure, may be detected by the capacitive sensor.

Additionally, and/or alternatively, further effects, which are induced by the person's body while sitting in the aircraft seat and/or while lying in the aircraft bed, may also interact with the electrostatic field generated by the capacitive sensor and may thus also be detected by the capacitive sensor, such bioelectric effects of the person's body, e.g., an electrocardiogram.

As mentioned above, capacitive sensing in general is known in the art.

However, capacitive sensing for use in-flight on an aircraft is neither known nor suggested in the prior art, let alone for sensing at least one physiological value of a person, when the person is arranged on at least a portion of an aircraft seat and/or aircraft bed, by a capacitive sensor integrated in the aircraft seat and/or the aircraft bed.

The physiological values/physiological data which the capacitive sensor may be configured to sense include, but are not limited to: respiratory rate, heart rate, blood pressure, sinus rhythm, and at least one bioelectrical value, preferably an electrocardiogram.

The system may be configured to determine values of one or more physiological parameters and/or physiological states based on the detected physiological data, e.g., by means of an internal or remote evaluating device or other processing means. The physiological parameters and/or physiological states, respectively, which may be determined include, but are not limited to, respiratory rate, heart rate, sinus rhythm, at least one bioelectrical value, preferably an electrocardiogram, intoxication, e.g., a blood alcohol concentration, wakefulness, level of anxiety, level of stress, and irregular breathing, e.g., hyperventilation. For instance, the system may be configured to access a plurality of predetermined indicators, preferably stored in an internal and/or external data storage, which provide an indication that certain detected physiological data correlate with a certain value or range of values of at least one physiological parameter and/or physiological state. The detected physiological data may be compared with the predetermined indicators and if the detected physiological data matches or is similar to one or more predetermined indicators then the value or range of values of at least one physiological parameter and/or physiological state which corresponds to the predetermined indicators may be determined and provided, e.g., to a storage and/or display. For instance, the predetermined indicators may be stored and accessed in the form of one or more look-up tables and/or algorithms.

Moreover, the capacitive sensor is further configured to generate at least one physiological signal based on the sensed physiological value.

The system may also include a transmitting device configured to transmit the physiological signal to a device, e.g., a device which is already integrated in the aircraft, for instance, as a part of an on-board entertainment system, or a separate receiving device, such as a user's wearable device, such as a smartwatch and/or smart glasses, and/or a mobile device, such as a tablet and/or a smartphone. In other words, the receiving device may be provided by the operators, e.g., the respective airline company, of the aircraft and/or the receiving device may also be a device which belongs to the user.

The transmitting device may be configured to be communicatively connected to the receiving device, either by a wire connection or wirelessly. For instance, the transmitting device may be configured to communicatively connect to the receiving device via a Bluetooth connection, preferably a Bluetooth Low Energy (BLE) connection. Other types of wireless connections may also be used. The transmitting device may also be connected to the receiving device via one or more cables.

In any case, the receiving device may be configured to process and/or evaluate the physiological signal and/or provide, for instance on a screen of the receiving device and/or a screen which is separate from the receiving device, and/or store physiological values and/or physiological parameters related to the person.

For this purpose, the receiving device may have a processing software installed thereon for processing and/or evaluating the physiological signal.

Thus, by detecting physiological data of a person in-flight in an aircraft, inter alia, by the at least one detecting device including at least one capacitive sensor integrated in the aircraft seat and/or the aircraft bed, respectively, physiological values of persons onboard on the aircraft inflight, i.e., the flight crew and/or the passengers, may be detected, and optionally also monitored.

As detailed at the beginning, due to the large distances covered during many aircraft flights, the in-flight time spent in the respective aircraft by the flight crew, e.g., the pilot(s) and/or flight attendants, and the passengers is often relatively long.

This may negatively impact a person's body and/or health. For instance, remaining in a certain position, such as a seating and/or lying position in an aircraft seat or an aircraft bed, respectively, for a prolonged period of time may result in a negative cardiovascular and/or respiratory disturbance response from the person's body. In particular, the inability, or at least reduced ability, to move, e.g., walk, in an aircraft may contribute to the negative impact which may occur when residing in an aircraft for a prolonged period of time, in particular when in a seated and/or lying position.

Moreover, in many cases the seating conditions in aircrafts, in particular the legroom provided to the person when seated, in particular in commercial aircrafts, may contribute to the negatives effect(s) on the person's body, e.g., to the person's cardiovascular and/or respiratory system.

Thus, detecting physiological data of a person in-flight in an aircraft, inter alia, by the at least one detecting device including at least one capacitive sensor integrated in the aircraft seat and/or the aircraft bed, respectively, may allow the general state of health and/or the general physiological state, e.g., awake, sleeping or unconscious, of persons in an aircraft inflight, such as the flight crew, e.g., the pilot and/or the flight attendants, and/or the passengers, to be detected, and optionally also monitored.

The system may monitor the physiological data, and optionally also provide warnings to the person whose physiological data is being detected and/or to other persons and/or to another device. For instance, the physiological data of a passenger may be detected and warnings may be sent the passenger and/or to the flight crew, e.g., to flight attendants.

Hence, by detecting physiological data of a person in-flight in an aircraft, and optionally also monitoring the physiological data, critical, or at least poor, health conditions of persons in an in-flight aircraft may be tended to, e.g., by the person whose physiological data is being detected and/or by other persons, such as onboard medical personnel and/or the flight crew.

Moreover, based on the detected physiological data, the person whose physiological data is being detected and/or by other persons may recognize that measures, such as by exercising, taking medication, changing their seating and/or lying position and/or seeking medical attention, should be taken to counteract worsening health conditions and/or health conditions which may worsen if no such measures are taken.

Thus, the system described herein for detecting physiological data of a person in-flight in an aircraft may prevent, or at least reduce, the risk of in-flight health-related complications which may occur.

Moreover, detecting physiological data of a person in-flight in an aircraft, inter alia, by the at least one detecting device including at least one capacitive sensor integrated in the aircraft seat and/or the aircraft bed, respectively, may give a general feedback to the person whose physiological data is being detected and/or by other persons regarding the general state of the person's health and/or their general physiological state, e.g., awake, sleeping or unconscious.

Nowadays, people are often generally interested in monitoring their own health, e.g., on a day to day basis, independently from medical personnel, e.g., doctors. Thus, for instance, providing information to the passengers regarding their general health based on physiological data detected by the system while in-flight in an aircraft, as described herein, may be a service which is desired and/or appreciated by the passengers and/or flight crew.

This may be particularly useful for people who travel in aircrafts frequently, e.g., to aid in assessing which effects travelling in aircrafts have on the person's health.

In particular, the operators of the aircraft, e.g., the airline companies, may offer such a service as an option, e.g., at an additional cost, in an aircraft, in particular to passengers.

Moreover, the system may allow the state of health of persons who are substantially, at least in part, responsible for the safety of the aircraft, such as the pilot(s) of the aircraft, to be determined and/or assessed, preferably on a regular basis.

Thus, this may allow the operators of the aircraft, e.g., the airline company, to provide medical attention to the concerned person, or at least prohibit the person from performing his/her duties which the safety of the aircraft substantially, at least in part, depends on.

Moreover, in case the detected physiological data of a pilot and/or a flight attendant shows that the pilot's and/or the flight attendant's health or general physiological state is poor and/or inappropriate for a current situation, e.g., the pilot and/or flight attendant may unconscious, tired, sleeping and/or drunk, this may be indicated to the pilot and/or flight attendant and/or other persons so that measures may be taken to secure the safety of the aircraft.

For instance, a second pilot on-board the aircraft may take over control of the aircraft or ground personnel may remotely take over the control of the aircraft.

The system may also indicate, via the physiological data, stress-related symptoms of the person, e.g., a pilot, whose physiological data is being detected.

Thus, the operators of the aircraft, e.g., the airline company, may take the physiological data showing stress-related symptoms into account when determining the type and/or amount of training and/or when creating new training for the pilot which may suitable to improve the pilot's reaction(s) to potentially stress-inducing in-flight situations in order to potentially reduce the stress-related symptoms of the pilot in the future.

Detecting the physiological data of the flight crew, e.g., the flight attendants and/or pilots, may allow a more frequent monitoring of the general health of the flight crew and/or their in-flight performance. This may be more effective and/or efficient than merely conducting yearly or half-yearly medical checks of the flight crew, in particular the pilot(s), by medical personnel.

The detected physiological data may also serve to generally determine whether a person is seated in an aircraft seat and/or is lying on an aircraft bed. This may be useful when the flight crew and/or the passengers are supposed to be seated in their respective aircraft seats or lying on their respective aircraft beds, e.g., during takeoff and/or landing and/or when turbulence occurs.

Determining whether a person is seated in an aircraft seat or is lying on an aircraft bed may also provide information whether the pilot(s) is/are seated in their respective aircraft seats to ensure the safety of the aircraft.

Alternatively, or additionally, the system may be configured to determine a seating position and/or a lying position of a person seated in an aircraft seat and/or lying on an aircraft bed based on the detected physiological data. Optionally, feedback information related to the determined seating position and/or lying position may be provided to the user by the system. The feedback information may include information to indicate the determined seating position and/or lying position to the user and/or information to indicate whether the determined seating position and/or lying position may be detrimental to the health of the user. The feedback information may optionally also provide at least one suggestion for assuming a healthier and/or less detrimental seating position and/or lying position. This may enable the system to prevent, or at least reduce, health-related complications of the user which may be caused by assuming an improper seating position and/or lying position.

The detected physiological data may also serve to recognize a potential threat to the safety of the aircraft by an on-board person or group of persons. For instance, a person or a group of persons who may be planning an attack on an aircraft may be detected since the physiological values, in particular the heart beat and/or blood pressure, of that person or that group of persons tend to become abnormal, in particular elevated, prior to the attack.

Thus, by detecting such abnormal, in particular elevated, physiological values, such a person or group of persons may be detected such that a threat to the aircraft may be recognized early in order to potentially prevent the attack.

The detected physiological data of one or all persons on-board the aircraft may be stored in an on-board storage and/or in a flight recorder (black box) of the aircraft. This may allow the stored physiological data to be assessed at a later time, e.g., after the flight has ended.

The detected physiological data may also be transmitted, e.g., via a transmitting device, to the ground, e.g., to a ground monitoring station. This may allow the physiological data to be monitored and/or assessed by people, e.g., ground personnel, on the ground rather than merely in the aircraft. This may, for instance, allow ground personnel to remotely take over the control of the aircraft quickly when critical and/or abnormal physiological data of a person or persons on-board the aircraft, in particular the pilot(s), is detected.

The system may further include at least one power source, e.g., one or more batteries, to provide electrical energy to the system. Alternatively, the system may be connected to and powered by an already present aircraft electrical system.

Preferably, the capacitive sensor includes at least two electrodes, preferably flat electrodes.

The at least two electrodes, preferably in the form of flat electrodes, may facilitate the integration of the capacitive sensor in or on the aircraft seat and/or aircraft bed. In particular, the at least two electrodes, preferably in the form of flat electrodes, may be non-intrusive, or only minimally intrusive, compared with other configurations of capacitive sensor.

Thus, the seating and/or lying comfort for a person sitting in the aircraft seat or lying on the aircraft bed, respectively, is not, or only minimally comprised, when the capacitive sensor is integrated thereon or therein. Thus, a high level of comfort to the person sitting or lying in the aircraft seat or the aircraft bed, respectively, may be maintained despite the integration of the capacitive sensor(s).

The at least two electrodes may be integrated within at least one cushion and/or underneath at least one cover of the aircraft seat and/or the aircraft bed.

For this purpose, the aircraft seat and/or the aircraft bed may include an accessing element configured to allow access to the capacitive sensor(s) integrated in the aircraft seat and/or aircraft bed.

Thus, the accessing element may enable the at least two electrodes to be accessed in order to, e.g., service the electrodes and/or replace the electrodes, e.g., when at least one electrode is faulty. This may facilitate servicing efforts of the system and thus reduce costs.

The accessing element may be, e.g., a removable portion of the aircraft seat and/or aircraft bed, e.g., a removable seat or bead cover, such as a removable seat or bead upholstery, or a removable portion of a cushion of the aircraft seat and/or aircraft bed.

The accessing element may also be a generally movable element, e.g., a swivelable and/or rotatable and/or slidable element, such as a flap or a sliding element, which may be configured to allow access to the electrodes by moving the movable element from a closed position, in which the movable element does not allow access to the respective electrode(s), to an access position, in which the movable element allows access to the respective electrode(s).

The electrodes may be arranged on an outer surface of the aircraft seat, e.g., on an outer surface of the seating portion and/or the backrest and/or the armrest(s) and/or the headrest(s), or on an outer surface of the aircraft bed, e.g., on an outer surface of the lying portion and/or the headrest(s).

Additionally, or alternatively, the electrodes may be arranged within the aircraft seat and/or aircraft bed, e.g., within a cushion of the seating portion and/or the backrest and/or the armrest(s) and/or the headrest(s) of the aircraft seat and/or within a cushion of the lying portion and/or the headrest(s) of the aircraft bed. The electrodes may also be arranged underneath an outer surface of the aircraft seat and/or the aircraft bed, such as underneath a seat cover or bed cover, e.g., a seat and/or bed upholstery.

In case the system includes at least one aircraft seat, the capacitive sensor is preferably arranged in or on a seating portion and/or in or on a backrest of the aircraft seat.

Such an arrangement may allow the capacitive sensorto be located proximate to the body, in particular to the torso, of the person whose physiological data is to be detected. This may facilitate detection of the physiological data and/or provide a more precise detection of said physiological data, in particular with respect to physiological data related to the cardiovascular and/or respiratory system of the person.

Preferably, the detecting device includes at least one transmitting device configured to connect, preferably wirelessly, to a receiving device, preferably to a mobile and/or wearable device, and to transmit the physiological data to the receiving device.

For instance, the transmitting device may be configured to communicatively connect to the receiving device via a Bluetooth connection, preferably a Bluetooth Low Energy (BLE) connection. Other types of wireless connections may also be used. The transmitting device may also be connected to the receiving device via one or more cables.

The transmitting device may be integrated in the detecting device and/or with other components of the system, such as those components described above or below, to form a single coherent unit with the detecting device, i.e. as one module. Alternatively, the transmitting device may be configured as a unit which is separate from, e.g., not fixedly attached to, the detecting device or any other component of the system.

The transmitting device may be configured to communicatively connect to the receiving device provided by the operators, e.g., the respective airline company, of the aircraft. For instance, the receiving device may be a device which is already integrated in the aircraft, for instance, as a part of an on-board entertainment system.

Alternatively, or additionally, the receiving device may be a separate receiving device, preferably a device which may be used for further functions other than merely other receiving data from the transmitting device, such as a user's smartphone.

In particular, the receiving device may be a device which belongs to the user, such as a wearable device, such as a smartwatch and/or smart glasses, and/or a mobile device, such as a tablet and/or a smartphone.

In other words, the receiving device may be provided by the operators, e.g., the respective airline company, of the aircraft and/or the receiving device may also be a device which belongs to the user.

As detailed at the beginning, the capacitive sensor generates an electrostatic field for detecting physiological data of a person's body sitting in the aircraft seat and/or lying on the aircraft bed.

As detailed above, capacitive sensing in general is known in the art.

However, capacitive sensing for use in-flight on an aircraft is not known or suggested in the prior art, let alone for sensing at least one physiological value of a person, when the person is arranged on at least a portion of an aircraft seat or aircraft bed, by a capacitive sensor integrated in the aircraft seat and/or the aircraft bed.

Hence, the system disclosed herein may be faced with challenges which have not been addressed in the prior art, in particular due to the lack of use of capacitive sensing on an in-flight aircraft in the prior art.

Various devices provided in an aircraft may generate electromagnetic fields, e.g., in the cabin and/or in the cockpit of the aircraft, which may cause interference with the electrostatic field generated by the capacitive sensor. For instance, the various flight control systems of an aircraft may generate such electromagnetic fields.

This interference may influence the physiological signal generated by the capacitive sensor.

To counteract these effects, the system includes a signal processing unit configured to process and/or adjust the physiological signal at least based on one or more electromagnetic interference signals, which are present in the environment of the system.

Thus, the signal processing unit may be configured to adapt the physiological signal to the environment in the aircraft in order to provide more reliable and/or more accurate physiological signals.

The signal processing unit may be arranged within the aircraft seat and/or aircraft bed, e.g., within a cushion of the seating portion and/or the backrest and/or the armrest(s) and/or the headrest(s) of the aircraft seat and/or within a cushion of the lying portion and/or the headrest(s) of the aircraft bed, to provide a relatively uncluttered and stowed away arrangement of the signal processing unit. Alternatively, the signal processing unit may be arranged elsewhere, such as underneath the aircraft seat and/or aircraft bed.

The signal processing unit may be integrated with the detecting device and/or with other components of the system, such as those components described above or below, to form a single coherent module. Alternatively, the signal processing unit may be configured as a unit which is separate from, e.g., not fixedly attached to, the detecting device or any other component of the system.

Preferably, the system further comprises an amplifying unit configured to amplify the physiological signal generated by the capacitive sensor and provide the amplified physiological signal to the signal processing unit such that the signal processing unit can process and/or adjust the amplified physiological signal.

In particular by amplifying the physiological signal generated by the capacitive sensor at the capacitive sensor, i.e., prior to the physiological signal being provided to the signal processing unit, the effect(s) of interference signals, e.g., electromagnetic interference signals, on the physiological signal may be reduced.

In particular physiological signals based on bioelectrical values detected from a person may be particularly susceptible to interference by interference signals, e.g., electromagnetic interference signals.

Thus, by amplifying such physiological signals prior to the physiological signals being provided to the signal processing unit, the effect(s) of interference signals on the physiological signals may be reduced.

This may allow bioelectrical values, preferably an electrocardiogram, of the person whose physiological data is to be detected, to be detected of a medical quality or at least of a quality which is sufficient enough to give a reliable indication of the state, e.g., good or poor, of the bioelectrical values.

Preferably, the detecting device is configured to apply analog and/or digital frequency-selective filtering to the physiological signal at least based on the interference signals.

By specifically adapting the physiological signal via analog and/or digital frequency-selective filtering at least based on the interference signals, the physiological signal may be specifically adapted to the environment in the aircraft in order to provide more reliable and/or more accurate physiological signals

Preferably, the detecting device comprises at least one passive electronic component, preferably at least one capacitor and/or at least one coil and/or at least one resistance, for performing analog frequency-selective filtering.

The above-identified passive electronic components may provide a reliable and/or efficient and/or effective means for specifically adapting the physiological signal to the environment in the aircraft in order to provide more reliable and/or more accurate physiological signals

Preferably, the system further comprises at least one operational amplifier for performing analog frequency-selective filtering.

The following devices and/or components, alternatively or additionally, may provide a reliable and/or efficient and/or effective means for specifically adapting the physiological signal to the environment in the aircraft in order to provide more reliable and/or more accurate physiological signals.

Preferably, the detecting device comprises at least one logic module, preferably at least one Application-Specific Integrated Circuit (ASIC) and/or at least one Field Programmable Gate Array (FPGA), for performing digital frequency-selective filtering.

Preferably, the signal processing unit is configured to detect one or more interference signals, at least with respect to at least one pulse width and/or at least one frequency of the interference signals, and filter the interference signals from the physiological signal and/or disregard the interference signals.

Preferably, the signal processing unit, for the purpose of applying frequency-selective filtering to the physiological signal, is configured to apply at least one Fourier-analysis and/or at least one autocorrelation and/or at least one adaptive algorithm to the physiological signal.

Preferably, the detecting device is configured to adjust, preferably smooth, a supply voltage provided by an aircraft electrical system to the detecting device.

An aircraft electrical system of an aircraft, in particular a commercial passenger and/or cargo airplane, typically generates an alternating current, which is sinusoidal and thus not constant.

Thus, adjusting, preferably smoothing, a supply voltage provided by an aircraft electrical system to the detecting device may enable a more reliable and/or more accurate physiological signal to be generated.

Preferably, the detecting device is configured to correct movement artifacts in the physiological signal caused by movement, preferably vibrations, present in the environment of the system by filtering and/or disregarding, preferably by at least one algorithm, effects in the physiological signal which are caused by the movement.

The environment in an in-flight aircraft is often influenced by movements, such as vibrations, having various causes. For instance, turbulence effected by the ambient outside of the aircraft may cause movements, e.g., tremors, within the aircraft. Moreover, the engines(s) of the aircraft may also cause movements within the aircraft. Such movements may cause movement artifacts in the physiological signal, which may reduce the accuracy and/or validity of the physiological signal.

Thus, by correcting such movement artifacts in the physiological signal caused by movement, preferably vibrations, present in the environment of the system by filtering and/or disregarding these effects in the physiological signal, a more reliable and/or more accurate physiological signal may be generated.

Preferably, the system further comprises an accelerometer and/or a gyroscope for detecting movement, preferably vibrations, present in the environment of the system to correct movement artifacts in the physiological signal caused by the movement.

The accelerometer and/or a gyroscope may be integrated in a portion of the aircraft seat and/or the aircraft bed, respectively. Alternatively, the accelerometer and/or a gyroscope may also be arranged outside of the aircraft seat and/or the aircraft bed, e.g., underneath the aircraft seat and/or aircraft bed. For the purpose of information exchange, the accelerometer and/or gyroscope may be communicatively coupled, preferably wirelessly, with a further component of the system, preferably the signal processing unit, which may then correct movement artifacts in the physiological signal caused by the movement detected by the movement detection device based on information provided by the accelerometer and/or gyroscope.

Preferably, the system further comprises shielding means configured to actively and/or passively shield at least portions of the system from interference signals, preferably electromagnetic interference signals, which are present in the environment of the system.

By providing such shielding means, for one, the effects of the interference signals on the generated physiological signals may be reduced or prevented as a whole. Moreover, the influence which the system described herein may have on other systems, such as flight control systems of the aircraft, may be reduced or even prevented. For instance, the physiological signals generated by the system described herein and/or the electrostatic field generated by the capacitive sensor may have a negative impact on other systems, such as flight control systems, of the aircraft.

The shielding means may comprise a material which is impermeable, or at least has a reduced permeability, to interference signals, preferably electromagnetic signals, to protect at least a portion of the system from the interference signals.

The shielding means may be a coating covering at least a portion of at least one of the components of the system, e.g., the amplifying unit, the signal processing unit or cables.

Alternatively, the shielding means may be a housing covering at least a portion of the system.

Preferably, the shielding means includes at least one layer of shielding material, preferably integrated within the aircraft seat or the aircraft bed, respectively, which is arranged between one or more sources of interference signals and at least a portion of the system to passively shield at least a portion of the system from interference signals.

Preferably, the shielding means includes at least one layer of shielding material provided around at least a portion of at least some of the cables of the system to passively shield at least a portion of the system from interference signals.

Preferably, the system is configured to detect at least one bioelectrical value of the person.

Preferably, the system is configured to detect physiological data related to at least one of the following of the person: respiratory rate, heart rate, sinus rhythm, and at least one bioelectrical value, preferably an electrocardiogram.

The system may be configured to determine one or more physiological parameters and/or physiological states based on the detected physiological data, e.g., by means of an internal or remote evaluating device or other processing means. The physiological parameters and/or physiological states, respectively, which may be determined include, but are not limited to, respiratory rate, heart rate, sinus rhythm, at least one bioelectrical value, preferably an electrocardiogram, intoxication, e.g., a blood alcohol concentration, wakefulness, level of anxiety, level of stress, and irregular breathing, e.g., hyperventilation. For instance, the system may be configured to access a plurality of predetermined indicators, preferably stored in an internal and/or external data storage, which provide an indication that certain detected physiological data correlate with a certain value or range of values of at least one physiological parameter and/or physiological state. The detected physiological data may be compared with the predetermined indicators and if the detected physiological data matches or is similar to one or more predetermined indicators then the value or range of values of at least one physiological parameter and/or physiological state which corresponds to the predetermined indicators may be determined and provided, e.g., to a storage and/or display. For instance, the predetermined indicators may be stored and accessed in the form of one or more look-up tables and/or algorithms.

Preferably, the capacitive sensor has a maximum total weight of 30 g, preferably 25 g, more preferably 20 g.

Limiting the on-board weight of aircrafts is generally desired, e.g., to reduce operational costs, such as fuel consumption. Hence, by limiting the maximum weight of the capacitive sensor to the above-identified values, the additional on-board weight caused by the capacitive sensor may be kept at a minimum.

The object set out at the beginning is also solved by a kit for in-flight monitoring of at least one physiological parameter of a person in-flight in an aircraft according to a second aspect of the invention.

The kit includes a system for detecting physiological data of a person in-flight in an aircraft, the system having any of the features and/or configurations described herein.

The kit further includes an evaluating device configured to determine, and optionally display, the physiological parameter of the person based on the physiological signal generated by the capacitive sensor.

Preferably, the evaluating device is one of the following: a mobile and/or wearable device of the user, a laptop, a pc, a tablet and an on-board entertainment system including at least one display for displaying the physiological parameter.

The features, configurations and/or advantages described above in relation to the system according to the first aspect of the invention also apply to the kit according to the second aspect of the invention accordingly.

Preferred embodiments of the present invention are further elucidated below with reference to the figures.
- Fig. 1: shows, in a side view, a system according to a first aspect and a kit according to a second aspect of the invention, including an aircraft seat;
- Fig. 2: shows, in a side view, a system according to a first aspect and a kit according to a second aspect of the invention, including an aircraft bed.

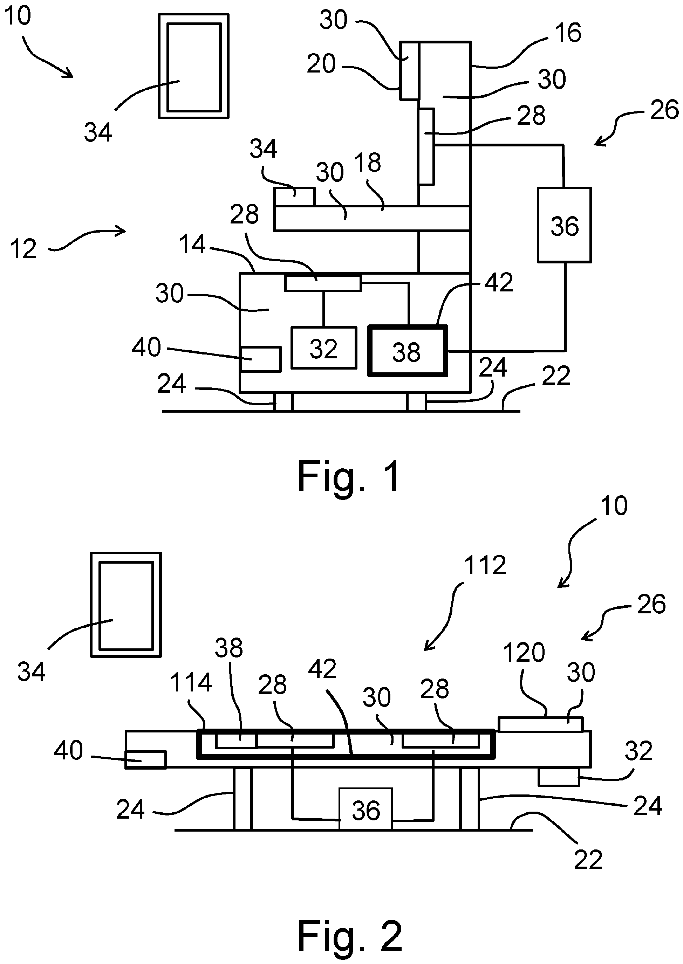

Figs. 1 and 2 show a system 10 for detecting physiological data of a person in-flight in an aircraft. In the embodiment shown in Fig. 1, the system 10 includes an aircraft seat 12 installed in an unpowered aircraft, such as a glider, or a powered aircraft, such as propeller aircraft or a jet aircraft. In contrast to Fig. 1, the system 10 shown in Fig. 2 includes an aircraft bed 112 instead of the aircraft seat 12 shown in Fig. 1.

The systems 10 shown in Figs. 1 and 2 may be identical, other than the system 10 shown in Fig. 1 including an aircraft seat 12 and the system 10 shown in Fig. 2 including an aircraft bed 112. The systems shown in Figs. 1 and 2 may also differ from each other in other ways. For instance, the components of the systems 10 shown in Fig. 1 and 2, respectively, do not have to be the same.

In addition, the system 10 may include both an aircraft seat 12, e.g., as shown in Fig. 1, and an aircraft bed 112, e.g., as shown in Fig. 2. The system 10 may also include a plurality of aircraft seats 12 and/or a plurality of aircraft beds 112.

The aircraft seat 12 and/or aircraft bed 112 may be arranged anywhere in the aircraft, e.g., in a cabin of the aircraft, as a seat or bed for a passenger or a flight attendant, or in a cockpit of the aircraft, as a seat or bed for a pilot of the aircraft.

The aircraft seat 12 shown in Fig. 1 includes a seating portion 14 configured for a person to sit on, a backrest 16, at least one armrest 18 and a headrest 20.

Moreover, the aircraft seat 12 is fixedly mounted to a floor 22 of the aircraft via mounting elements 24.

The aircraft bed 112 shown in Fig. 2 includes a lying portion 114 configured for a person to lie on and a headrest 120.

The system 10 further includes a detecting device 26 including capacitive sensors 28. Figs. 1 and 2 each show a plurality of capacitive sensors 28. The system 10 may have any number of capacitive sensors 28. Alternatively, the system 10 may include only a single capacitive sensor 28.

The plurality of capacitive sensors 28 may be a part of a single detecting device 26. Alternatively, each capacitive sensor 28 may be associated with its own detecting device 26

The seating portion 14 and the backrest 16 are each provided with one capacitive sensor 28 integrated therein, respectively, as shown in Fig 1. Moreover, the armrest 18 and/or the headrest 20 may also be provided with one or more capacitive sensors 28.

Thus, the seating portion 14 and/or the armrest(s) 18 and/or the backrest 16 and/or the headrest 20 may each be provided with one or more one capacitive sensors 28.

The capacitive sensor(s) 28 may be arranged on an outer surface of the aircraft seat 12, e.g., on an outer surface of the seating portion 14 and/or the armrest(s) 18 and/or the backrest 16 and/or the headrest 20.

Additionally, or alternatively, the capacitive sensor(s) may be arranged within the aircraft seat 12, e.g., within a cushion 30 of the seating portion 14 and/or the armrest(s) 18 and/or the backrest 16 and/or the headrest 20. The capacitive sensor 28 may also be arranged underneath an outer surface of the aircraft seat 12, such as underneath a seat cover, e.g., a seat upholstery, which is not specifically shown in Fig. 1.

In the case of the aircraft bed 112 shown in Fig. 2, the lying portion 114 is provided with two capacitive sensors 28 integrated therein, as shown in Fig 2. Moreover, the headrest 120 may also be provided with one or more capacitive sensors 28.

Thus, the lying portion 114 and/or the headrest 120 may each be provided with one or more one capacitive sensors 28.

The capacitive sensor(s) 28 may be arranged on an outer surface of the aircraft bed 112, e.g., on an outer surface of the lying portion 114 and/or the headrest 120.

Additionally, or alternatively, the capacitive sensor(s) may be arranged within the aircraft bed 112, e.g., within a cushion 30 of the lying portion 114 and/or the headrest 120. The capacitive sensor 28 may also be arranged underneath an outer surface of the aircraft bed 112, such as underneath a seat cover, e.g., a seat upholstery, which is not specifically shown in Fig. 2.

The capacitive sensor 28 is configured to sense at least one physiological value of a person, when the person is arranged on at least a portion of the aircraft seat 12, or, in the case of an aircraft bed 112, on at least a portion of the aircraft bed 112. The capacitive sensor 28 is further configured to generate at least one physiological signal based on the sensed physiological value.

The physiological data/values which may be detected/sensed may include, but are not limited to, respiratory rate, heart rate, sinus rhythm, and at least one bioelectrical value, preferably an electrocardiogram.

The system 10 may be configured to determine one or more physiological parameters and/or physiological states based on the detected physiological data, e.g., by means of an internal or remote evaluating device or other processing means. The physiological parameters and/or physiological states, respectively, which may be determined include, but are not limited to, respiratory rate, heart rate, sinus rhythm, at least one bioelectrical value, preferably an electrocardiogram, intoxication, e.g., a blood alcohol concentration, wakefulness, level of anxiety, level of stress, and irregular breathing, e.g., hyperventilation. For instance, the system 10 may be configured to access a plurality of predetermined indicators, preferably stored in an internal and/or external data storage, which provide an indication that certain detected physiological data correlate with a certain value or range of values of at least one physiological parameter and/or physiological state. The detected physiological data may be compared with the predetermined indicators and if the detected physiological data matches or is similar to one or more predetermined indicators then the value or range of values of at least one physiological parameter and/or physiological state which corresponds to the predetermined indicators may be determined and provided, e.g., to a storage and/or display. For instance, the predetermined indicators may be stored and accessed in the form of one or more look-up tables and/or algorithms.

The capacitive sensor 28 may be configured to sense one or more physiological values of the person in a non-contact manner. In other words, the person's body does not have to contact the capacitive sensor 28 in order for the capacitive sensor 28 to sense the physiological value of the person. Instead, the capacitive sensor 28 may sense one or more physiological values when the person's body is proximate to the capacitive sensor 28, e.g., when the person is at least partially seated in the aircraft seat 12 or at least partially lying on the aircraft bed 112.

Thus, movement of the person while sitting in the aircraft seat 12 or lying on the aircraft bed 112, such as movements caused by, but not limited to, the person's breathing and /or heart beat and/or blood pressure, may be detected by the capacitive sensor 28.

Additionally, and/or alternatively, further effects, which may be induced by the person's body while sitting in the aircraft seat 12 or lying on the aircraft bed 112, may also interact with the electrostatic field generated by the capacitive sensor 28 and may thus also be detected by the capacitive sensor 28, such bioelectric effects of the person's body, e.g., an electrocardiogram.

By detecting physiological data of a person in-flight in an aircraft, inter alia, by the at least one detecting device 26 including at least one capacitive sensor 28 integrated in the aircraft seat 12 and/or the aircraft bed 112, as shown in Figs. 1 and 2, physiological values of persons onboard the in-flight aircraft, i.e., the flight crew and/or the passengers, may be detected, and optionally also monitored.

There are a number of benefits of detecting physiological data of a person in-flight in an aircraft, as detailed at the beginning.

For instance, travelling long distances in aircrafts may negatively impact a persons' body and/or health.

Thus, by providing the system 10 described herein for detecting physiological data of a person in-flight in an aircraft may prevent, or at least reduce, the risk of in-flight health-related complications which may potentially occur.

Moreover, the system 10 may allow a general state of health and/or a general physiological state, e.g., awake, sleeping or unconscious, of persons in an inflight aircraft, such as the flight crew, e.g., the pilot and/or the flight attendants, and/or the passengers, to be detected, and optionally also monitored.

In addition, the system 10 may be particularly useful for people who travel in aircrafts frequently, e.g., to aid in assessing which effects travelling in aircrafts have on the person's body and/ or health, in particular the person's cardiovascular system and/or respiratory system.

In particular, the operators of the aircraft, e.g., the airline companies, may offer such a service as an option, e.g., at an additional cost, in an aircraft, in particular to passengers of commercial flights.

The detected physiological data may also serve to generally determine whether a person is seated in the aircraft seat 12 or is lying on the aircraft bed 112. This may be useful when the flight crew and/or the passengers are supposed to be seated in their respective aircraft seats 12 and/or lying on their respective aircraft beds 112, e.g., during takeoff and/or landing and/or when turbulence occurs.

Determining whether a person is seated in the aircraft seat 12 and/or is lying on the aircraft bed 112 may also provide information whether the pilot(s) is/are seated in their respective aircraft seat 12 or on their respective bed 112 to ensure the safety of the aircraft and/or the safety of the persons.

The system 10 may include a storage for storing the detected physiological data of one or all persons on-board the aircraft. Alternatively, the physiological data may be stored in a separate on-board storage and/or in a flight recorder (black box) of the aircraft. This may allow the stored physiological data to be assessed at a later time, e.g., after the flight has ended.

Preferably, the capacitive sensor 28 includes at least two electrodes, preferably flat electrodes, which are integrated within at least one cushion 30 and/or underneath at least one cover of the aircraft seat 12.

The detecting device 26 includes at least one transmitting device 32 configured to connect, preferably wirelessly, to a receiving device 34, preferably to a mobile and/or wearable device, and to transmit the physiological data to the receiving device 34.

The transmitting device 32 may be arranged anywhere in the aircraft, e.g., within the aircraft seat 12 (see Fig. 1) and/or the aircraft bed 112, on a surface of the aircraft seat 12 and/or the aircraft bed 112 (see Fig. 1) or underneath the aircraft seat 12 and/or the aircraft bed 112.

For instance, the transmitting device 32 may be configured to communicatively connect to the receiving device 34 via a Bluetooth connection, preferably a Bluetooth Low Energy (BLE) connection. Other types of wireless connection are also feasible. The transmitting device 32 may also be connected to the receiving device 34 via one or more cables.

For instance, the transmitting device 32 may be configured to communicatively connect to a receiving device 34 provided by the operators, e.g., the respective airline company, of the aircraft. For instance, such a receiving device 34 may be part of an on-board entertainment system of the aircraft, e.g., attached to the armrest 18 of the aircraft chair 12, as shown in Fig. 1, or attached to a backside of the aircraft seat 12 located in front of the aircraft seat 12 shown in Fig. 1.

Alternatively, or additionally, the receiving device 34 may be a separate device, e.g., which belongs to the user, such as a wearable device, such as a smartwatch and/or smart glasses, and/or a mobile device, such as a tablet and/or a smartphone, as also shown in Fig. 1 by the receiving device 34 in the upper left hand corner of Fig. 1.

In any case, the receiving device 34 may be configured to process and/or evaluate the physiological signal and/or provide physiological parameters related to the person seated om the aircraft seat 12 and/or lying on the aircraft bed 112, for instance on an integrated screen of the receiving device 34 and/or a screen which is separate from the receiving device 34.

For this purpose, the receiving device 34 may have a processing software installed thereon for processing and/or evaluating, and optionally also storing, the physiological signal.

The detected physiological data may also be transmitted, e.g., via the transmitting device 32, to the ground, e.g., to a ground monitoring station. This may allow the physiological data to be monitored and/or assessed by people, e.g., ground personnel, on the ground rather than merely in the aircraft. This may allow ground personnel to remotely take over the control of the aircraft quickly, e.g., when critical and/or abnormal physiological data of the pilot(s) is detected.

Thus, the receiving device 34 may also be provided on the ground, e.g., in a ground monitoring station. At least a first receiving device 34 may be provided on the ground and at least a second receiving device 34 may be provided in the aircraft.

Capacitive sensing for use in-flight on an aircraft may be faced with various challenges related to the environment in the aircraft.

For instance, various devices provided in an aircraft may generate electromagnetic fields, e.g., in the cabin and/or in the cockpit of the aircraft, which may cause interference with the electrostatic field generated by the capacitive sensor 28.

This interference may influence the physiological signal generated by the capacitive sensor 28.

To counteract these effects, the system 10 includes a signal processing unit 36 configured to process and/or adjust the physiological signal at least based on one or more electromagnetic interference signals, which may be present in the environment in the aircraft.

Thus, the signal processing unit 36 may be configured to adapt the physiological signal to the environment in the aircraft in order to provide more reliable and/or more accurate physiological signals.

The signal processing unit 36 may be arranged anywhere in the aircraft, e.g., within the aircraft seat 12 and/or the aircraft bed 112, on a surface of the aircraft seat 12 and/or the aircraft bed 112 or underneath the aircraft seat 12 and/or the aircraft bed 112. The signal processing unit 36 is shown as being arranged outside of the aircraft seat 12 and the aircraft bed 112. However, the location of the signal processing unit 36 is only shown schematically in Figs. 1 and 2. As detailed above, the signal processing unit 36 may be arranged anywhere in the aircraft.

The system 10 may further comprise an amplifying unit 38 configured to amplify the physiological signal generated by the capacitive sensor 28 and provide the amplified physiological signal to the signal processing unit 36 such that the signal processing unit 36 can process and/or adjust the amplified physiological signal.

In particular by amplifying the physiological signal generated by the capacitive sensor 28 at the capacitive sensor 28, i.e., prior to the physiological signal being provided to the signal processing unit, the effect(s) of interference signals, e.g., electromagnetic interference signals, on the physiological signal may be reduced.

For instance, physiological signals based on bioelectrical values detected from a person may be particularly susceptible to interference by interference signals, e.g., electromagnetic interference signals.

Thus, by amplifying such physiological signals prior to the physiological signals being provided to the signal processing unit 36, the effect(s) of interference signals on the physiological signals may be reduced.

The amplifying unit 38 may be arranged anywhere in the aircraft, e.g., within the aircraft seat 12 (see Fig. 1) and/or the aircraft bed 112 (see Fig. 2), on a surface of the aircraft seat 12 and/or the aircraft bed 112, or underneath the aircraft seat 12 and/or the aircraft bed 112.

The amplifying unit 38 is shown as being arranged within the aircraft seat 12 and the aircraft bed 112, respectively, in Figs. 1 and 2. While such an arrangement may provide a relatively uncluttered and stowed away arrangement of the amplifying unit 38, the arrangement of the amplifying unit 38 is only shown schematically in Figs. 1 and 2. As detailed above, the amplifying unit 38 may be arranged anywhere in the aircraft.

The system 10 may include one or more means, as described below, for enhancing the quality of the physiological signal, in particular with regard to the environment in the aircraft in order to provide more reliable and/or more accurate physiological signals.

For instance, the detecting device 26 may be configured to apply analog and/or digital frequency-selective filtering to the physiological signal at least based on the interference signals.

Moreover, at least one passive electronic component, preferably at least one capacitor and/or at least one coil and/or at least one resistance, may be provided for performing analog frequency-selective filtering.

In addition, the system 10 may further comprise at least one operational amplifier for performing analog frequency-selective filtering.

Furthermore, at least one logic module, preferably at least one Application-Specific Integrated Circuit (ASIC) and/or at least one Field Programmable Gate Array (FPGA), may be provided for performing digital frequency-selective filtering.

Preferably, the signal processing unit 36 is configured to detect one or more interference signals, at least with respect to at least one pulse width and/or at least one frequency of the interference signals, and filter the interference signals from the physiological signal and/or disregard the interference signals.

Preferably, the signal processing unit 36, for the purpose of applying frequency-selective filtering to the physiological signal, is configured to apply at least one Fourier-analysis and/or at least one autocorrelation and/or at least one adaptive algorithm to the physiological signal.

The environment in an in-flight aircraft is often influenced by movements, such as vibrations, having various causes. For instance, turbulence effected by the ambient outside of the aircraft may cause movements, e.g., tremors, within the aircraft. Moreover, the engines(s) of the aircraft may also cause movements within the aircraft. Such movements may cause movement artifacts in the physiological signal.

Thus, the detecting device 26 may be configured to correct movement artifacts in the physiological signal caused by movement, preferably vibrations, present in the environment of the system by filtering and/or disregarding, preferably by at least one algorithm, effects in the physiological signal which are caused by the movement.

By correcting such movement artifacts in the physiological signal caused by movement, preferably vibrations, present in the environment of the system by filtering and/or disregarding these effects in the physiological signal, a more reliable and/or more accurate physiological signal may be generated.

The system 10 may further comprise at least one movement detection device 40, such as an accelerometer and/or a gyroscope, for detecting movement, preferably vibrations, present in the environment of the system 10 to correct movement artifacts in the physiological signal caused by the movement.

The movement detection device 40 may be integrated in a portion of the aircraft seat 12 and/or the aircraft bed 112, as shown in Figs. 1 and 2, respectively. Alternatively, the movement detection device 40 may also be arranged outside of the aircraft seat 12 and/or the aircraft bed 112, e.g., underneath the aircraft seat 12 and/or the aircraft bed 112, respectively.

The movement detection device 40 may be communicatively coupled, preferably wirelessly, with a further component of the system 10, preferably the signal processing unit 36, to exchange information. The further component of the system 10, preferably the signal processing unit 36, may then correct movement artifacts in the physiological signal caused by the movement detected by the movement detection device 40 based on the information provided by the movement detection device 40.

The system may further comprise shielding means 42 configured to actively and/or passively shield at least portions of the system 10 from interference signals, preferably electromagnetic interference signals, which are present in the environment of the system 10. The shielding means 42 are represented by bold lines in Figs. 1 and 2.

As shown in Fig. 1, the shielding means 42 may encompass only one of the components of the system 10. In the embodiment shown in Fig. 1, the shielding means 42 covers the amplifying unit 38.

The shielding means 42 may also encompass a plurality of the components of the system 10. In the embodiment shown in Fig. 1, the shielding means 42 covers the amplifying unit 38 and the capacitive sensor 28 arranged in the seating portion 14 of the aircraft seat 12.

The shielding means 42 may cover all of the components of the system 10, preferably all of the components of the system 10 which are particularly sensitive to interference signals such that said interference signals do not substantially influence the generated physiological signals or that the influence is at least reduced.

The shielding means 42 may comprise a material which is impermeable, or at least has a reduced permeability, to the interference signals, preferably electromagnetic signals, to protect at least a portion of the system 10 from the interference signals.

For instance, the shielding means 42 may at least partially comprise sheet metal, metal screen, and metal foam. The shielding means 42 may at least partially be made of copper, brass, nickel, silver, steel, and tin.

The shielding means 42 may include at least one layer of shielding material 42 integrated within the aircraft seat 12 or the aircraft bed 1121, respectively, as shown in Figs. 1 and 2. The shielding means 42 is preferably arranged between one or more sources of interference signals and at least a portion of the system 10 to passively shield at least a portion of the system 10 from interference signals.

The shielding means 42 may be a coating covering at least a portion of at least one of the components, e.g., the amplifying unit 38, the signal processing unit 36, cables and/or the capacitive sensors 28.

Alternatively, the shielding means 42 may be a housing covering at least a portion of the system 10, e.g., the amplifying unit 38, the signal processing unit 36, cables and/or the capacitive sensors 28.

The receiving device 34 described above may also be an evaluating device 34, which may be a component of a kit for in-flight monitoring of at least one physiological parameter of a person in-flight in an aircraft.

The kit includes the system 10 described herein. The evaluating device 34 may be configured to determine, and optionally display and/or store, one or more physiological parameters of the person based on the physiological signal generated by the capacitive sensor 28.

The evaluating device 34 may be one of the following: a mobile and/or wearable device of the user, a laptop, a pc, a tablet and an on-board entertainment system including at least one display for displaying the physiological parameter.

The system 10 may further include at least one power source, e.g., one or more batteries, to provide electrical energy to at least a portion of the system 10. Alternatively, the system 10 may be connected to and powered by an already present aircraft electrical system.

## Claims

1. A system (10) for detecting physiological data of a person in-flight in an aircraft, including:
- at least one aircraft seat (12) and/or at least one aircraft bed (112);
- at least one detecting device (26) including at least one capacitive sensor (28) integrated in the aircraft seat (12) and/or the aircraft bed (112), respectively, the capacitive sensor (28) being configured to sense at least one physiological value of a person, when the person is arranged on at least a portion of the aircraft seat (12) or aircraft bed (112), and the capacitive sensor (28) being further configured to generate at least one physiological signal based on the sensed physiological value; and
- a signal processing unit (36) configured to process and/or adjust the physiological signal at least based on one or more electromagnetic interference signals which are present in the environment of the system (10).

2. The system (10) according to claim 1, wherein, in case the system includes at least one aircraft seat (12), the capacitive sensor (28) is arranged in or on a seating portion (14) and/or in or on a backrest (16) of the aircraft seat (12).

3. The system (10) according to claims 1 or 2, wherein the detecting device (26) includes at least one transmitting device (32) configured to connect, preferably wirelessly, to a receiving device (34), preferably to a mobile and/or wearable device, and to transmit the physiological data to the receiving device (34).

4. The system (10) according to any of the preceding claims, further comprising an amplifying unit (38) configured to amplify the physiological signal generated by the capacitive sensor (28) and provide the amplified physiological signal to the signal processing unit (36) such that the signal processing unit (36) can process and/or adjust the amplified physiological signal.

5. The system (10) according to any of the preceding claims, wherein the detecting device (26) is configured to apply analog and/or digital frequency-selective filtering to the physiological signal at least based on the interference signals.

6. The system (10) according to claim 5, wherein the detecting device (26) comprises at least one passive electronic component, preferably at least one capacitor and/or at least one coil and/or at least one resistance, for performing analog frequency-selective filtering.

7. The system (10) according to claims 5 or 6, wherein the detecting device (26) comprises at least one logic module, preferably at least one Application-Specific Integrated Circuit (ASIC) and/or at least one Field Programmable Gate Array (FPGA), for performing digital frequency-selective filtering.

8. The system (10) according to any of the preceding claims, wherein the signal processing unit (36) is configured to detect one or more interference signals, at least with respect to at least one pulse width and/or at least one frequency of the interference signals, and filter the interference signals from the physiological signal and/or disregard the interference signals.

9. The system (10) according to any of the preceding claims, wherein the signal processing unit (36), for the purpose of applying frequency-selective filtering to the physiological signal, is configured to apply at least one Fourier-analysis and/or at least one autocorrelation and/or at least one adaptive algorithm to the physiological signal.

10. The system (10) according to any of the preceding claims, wherein the detecting device (26) is configured to correct movement artifacts in the physiological signal caused by movement, preferably vibrations, present in the environment of the system (10) by filtering and/or disregarding, preferably by at least one algorithm, effects in the physiological signal which are caused by the movement.

11. The system (10) according to any of the preceding claims, further comprising shielding means (42) configured to actively and/or passively shield at least portions of the system (10) from interference signals, preferably electromagnetic interference signals, which are present in the environment of the system (10).

12. The system (10) according to any of the preceding claims, wherein the system is configured to detect physiological data related to at least one of the following of the person: respiratory rate, heart rate, sinus rhythm, and at least one bioelectrical value, preferably an electrocardiogram.

13. A kit for in-flight monitoring of at least one physiological parameter of a person in-flight in an aircraft, including a system (10) according to any of the preceding claims and an evaluating device (34) configured to determine, and optionally display, the physiological parameter of the person based on the physiological signal generated by the capacitive sensor (28).

14. The kit according to claim 13, wherein the evaluating device (34) is one of the following: a mobile and/or wearable device of the user, a laptop, a pc, a tablet and an on-board entertainment system including at least one display for displaying the physiological parameter.

## Patentansprüche

1. System (10) zum Detektieren physiologischer Daten einer Person während eines Fluges in einem Flugzeug, aufweisend:
mindestens einen Flugzeugsitz (12) und/oder mindestens ein Flugzeugbett (112);
mindestens eine Detektionsvorrichtung (26), die mindestens einen kapazitiven Sensor (28) aufweist, der in den Flugzeugsitz (12) bzw. das Flugzeugbett (112) integriert ist, wobei der kapazitive Sensor (28) dazu eingerichtet ist, mindestens einen physiologischen Wert einer Person zu detektieren, wenn die Person auf mindestens einem Teil des Flugzeugsitzes (12) oder des Flugzeugbettes (112) angeordnet ist, und der kapazitive Sensor (28) ferner dazu eingerichtet ist, mindestens ein physiologisches Signal basierend auf dem detektierten physiologischen Wert zu generieren; und
eine Signalverarbeitungseinheit (36), die dazu eingerichtet ist, das physiologische Signal mindestens basierend auf einem oder mehreren elektromagnetischen Störsignalen, die in der Umgebung des Systems (10) vorhanden sind, zu verarbeiten und/oder einzustellen.

2. System (10) nach Anspruch 1, wobei, falls das System mindestens einen Flugzeugsitz (12) aufweist, der kapazitive Sensor (28) in oder an einem Sitzabschnitt (14) und/oder in oder an einer Rückenlehne (16) des Flugzeugsitzes (12) angeordnet ist.

3. System (10) nach Anspruch 1 oder 2, wobei die Detektionsvorrichtung (26) mindestens eine Sendevorrichtung (32) aufweist, die dazu eingerichtet ist, sich mit einer Empfangsvorrichtung (34), vorzugsweise mit einem mobilen und/oder tragbaren Gerät, zu verbinden, vorzugsweise drahtlos, und die physiologischen Daten an die Empfangsvorrichtung (34) zu senden.

4. System (10) nach einem der vorhergehenden Ansprüche, das ferner eine Verstärkungseinheit (38) aufweist, die dazu eingerichtet ist, das von dem kapazitiven Sensor (28) generierte physiologische Signal zu verstärken und das verstärkte physiologische Signal der Signalverarbeitungseinheit (36) bereitzustellen, so dass die Signalverarbeitungseinheit (36) das verstärkte physiologische Signal verarbeiten und/oder einstellen kann.

5. System (10) nach einem der vorhergehenden Ansprüche, wobei die Detektionsvorrichtung (26) dazu eingerichtet ist, eine analoge und/oder digitale frequenzselektive Filterung auf das physiologische Signal anzuwenden basierend mindestens auf den Störsignalen.

6. System (10) nach Anspruch 5, wobei die Detektionsvorrichtung (26) mindestens ein passives elektronisches Bauelement, vorzugsweise mindestens einen Kondensator und/oder mindestens eine Spule und/oder mindestens einen Widerstand, zur Durchführung einer analogen frequenzselektiven Filterung aufweist.

7. System (10) nach Anspruch 5 oder 6, wobei die Detektionsvorrichtung (26) mindestens ein Logikmodul, vorzugsweise mindestens eine anwendungsspezifische integrierte Schaltung (ASIC) und/oder mindestens ein feldprogrammierbares Gate-Array (FPGA), zur Durchführung einer digitalen frequenzselektiven Filterung aufweist.

8. System (10) nach einem der vorhergehenden Ansprüche, wobei die Signalverarbeitungseinheit (36) dazu eingerichtet ist, ein oder mehrere Störsignale mindestens in Bezug auf mindestens eine Pulsbreite und/oder mindestens eine Frequenz der Störsignale zu detektieren und die Störsignale aus dem physiologischen Signal zu filtern und/oder die Störsignale zu ignorieren.

9. System (10) nach einem der vorhergehenden Ansprüche, wobei die Signalverarbeitungseinheit (36) zum Zweck der Anwendung einer frequenzselektiven Filterung auf das physiologische Signal dazu eingerichtet ist, mindestens eine Fourier-Analyse und/oder mindestens eine Autokorrelation und/oder mindestens einen adaptiven Algorithmus auf das physiologische Signal anzuwenden.

10. System (10) nach einem der vorhergehenden Ansprüche, wobei die Detektionsvorrichtung (26) dazu eingerichtet ist, Bewegungsartefakte im physiologischen Signal zu korrigieren, die durch Bewegung, vorzugsweise Vibrationen, in der Umgebung des Systems (10) verursacht werden, durch Filtern oder Ignorieren, vorzugsweise durch mindestens einen Algorithmus, von Effekten im physiologischen Signal, die durch die Bewegung verursacht werden.

11. System (10) nach einem der vorhergehenden Ansprüche, das ferner eine Abschirmeinrichtung (42) aufweist, die dazu eingerichtet ist, mindestens Abschnitte des Systems (10) aktiv und/oder passiv von Störsignalen, vorzugsweise elektromagnetischen Störsignalen, abzuschirmen, die in der Umgebung des Systems (10) vorhanden sind.

12. System (10) nach einem der vorhergehenden Ansprüche, wobei das System dazu eingerichtet ist, physiologische Daten zu detektieren, die mit mindestens einem des Folgenden der Person zusammenhängen: Atemfrequenz, Herzfrequenz, Sinusrhythmus und mindestens einem bioelektrischen Wert, vorzugsweise einem Elektrokardiogramm.

13. Kit zur Flugüberwachung mindestens eines physiologischen Parameters einer Person während eines Fluges in einem Flugzeug, aufweisend ein System (10) nach einem der vorhergehenden Ansprüche und eine Auswertungsvorrichtung (34), die dazu eingerichtet ist, den physiologischen Parameter der Person basierend auf dem von dem kapazitiven Sensor (28) generierten physiologischen Signal zu bestimmen und optional anzuzeigen.

14. Kit nach Anspruch 13, wobei das Auswertegerät (34) eines der folgenden ist: ein mobiles und/oder tragbares Gerät des Benutzers, ein Laptop, ein PC, ein Tablet und ein Bordunterhaltungssystem, das mindestens eine Anzeige zum Anzeigen des physiologischen Parameters aufweist.

## Revendications

1. Système (10) de détection de données physiologiques d'une personne en vol dans un aéronef, comprenant :
au moins un siège d'aéronef (12) et/ou au moins un lit d'aéronef (112) ;
au moins un dispositif de détection (26) comprenant au moins un capteur capacitif (28) intégré dans le siège d'aéronef (12) et/ou le lit d'aéronef (112), respectivement, ledit capteur capacitif (28) étant configuré pour détecter au moins une valeur physiologique d'une personne, lorsque la personne est disposée sur au moins une partie du siège d'aéronef (12) ou du lit d'aéronef (112), et ledit capteur capacitif (28) étant en outre configuré pour générer au moins un signal physiologique sur la base de la valeur physiologique détectée ; et
une unité de traitement de signal (36) configurée pour traiter et/ou ajuster le signal physiologique au moins sur la base d'un ou de plusieurs signaux d'interférence électromagnétique présents dans l'environnement du système (10).

2. Système (10) selon la revendication 1, où, dans le cas où le système comprend au moins un siège d'aéronef (12), le capteur capacitif (28) est disposé dans ou sur une partie d'assise (14) et/ou dans ou sur un dossier (16) du siège d'aéronef (12).

3. Système (10) selon la revendication 1 ou la revendication 2, où le dispositif de détection (26) comprend au moins un dispositif de transmission (32) configuré pour se connecter, de préférence sans fil, à un dispositif de réception (34), de préférence à un dispositif mobile et/ou portable, et pour transmettre les données physiologiques au dispositif de réception (34).

4. Système (10) selon l'une des revendications précédentes, comprenant en outre une unité d'amplification (38) configurée pour amplifier le signal physiologique généré par le capteur capacitif (28) et délivrer le signal physiologique amplifié à l'unité de traitement de signal (36) de sorte que l'unité de traitement de signal (36) puisse traiter et/ou ajuster le signal physiologique amplifié.

5. Système (10) selon l'une des revendications précédentes, où le dispositif de détection (26) est configuré pour appliquer un filtrage analogique et/ou numérique sélectif en fréquence au signal physiologique au moins sur la base des signaux d'interférence.

6. Système (10) selon la revendication 5, où le dispositif de détection (26) comprend au moins un composant électronique passif, de préférence au moins un condensateur et/ou au moins une bobine et/ou au moins une résistance, pour effectuer un filtrage analogique sélectif en fréquence.

7. Système (10) selon la revendication 5 ou la revendication 6, où le dispositif de détection (26) comprend au moins un module logique, de préférence au moins un circuit intégré à application spécifique (ASIC) et/ou au moins une matrice prédiffusée programmable (FPGA), pour effectuer un filtrage numérique sélectif en fréquence.

8. Système (10) selon l'une des revendications précédentes, où l'unité de traitement de signal (36) est configurée pour détecter un ou plusieurs signaux d'interférence, au moins par rapport à au moins une largeur d'impulsion et/ou au moins une fréquence des signaux d'interférence, et filtrer les signaux d'interférence du signal physiologique et/ou négliger les signaux d'interférence.

9. Système (10) selon l'une des revendications précédentes, où, afin d'appliquer un filtrage sélectif en fréquence au signal physiologique, l'unité de traitement de signal (36) est configurée pour appliquer au signal physiologique au moins une analyse de Fourier et/ou au moins une autocorrélation et/ou au moins un algorithme adaptatif.

10. Système (10) selon l'une des revendications précédentes, où le dispositif de détection (26) est configuré pour corriger les artéfacts de mouvement dans le signal physiologique dus à des mouvements, de préférence à des vibrations, présents dans l'environnement du système (10), en filtrant et/ou en négligeant, de préférence par au moins un algorithme, les effets dus au mouvement dans le signal physiologique.

11. Système (10) selon l'une des revendications précédentes, comprenant en outre des moyens de blindage (42) configurés pour protéger activement et/ou passivement au moins des parties du système (10) contre des signaux d'interférence, de préférence des signaux d'interférence électromagnétiques, présents dans l'environnement du système (10).

12. Système (10) selon l'une des revendications précédentes, où le système est configuré pour détecter des données physiologiques relatives à au moins une des conditions suivantes de la personne : fréquence respiratoire, fréquence cardiaque, rythme sinusal, et au moins une valeur bioélectrique, de préférence un électrocardiogramme.

13. Kit de surveillance en vol d'au moins un paramètre physiologique d'une personne en vol dans un aéronef, comprenant un système (10) selon l'une des revendications précédentes et un dispositif d'évaluation (34) configuré pour déterminer, et éventuellement afficher, le paramètre physiologique de la personne sur la base du signal physiologique généré par le capteur capacitif (28).

14. Kit selon la revendication 13, où le dispositif d'évaluation (34) est l'un des suivants : un dispositif mobile et/ou portable de l'utilisateur, un ordinateur portable, un PC, une tablette ou un système audiovisuel embarqué comprenant au moins un écran pour afficher le paramètre physiologique.
